# EUROPÄISCHE PATENTSCHRIFT

(11) **EP 1 234 879 B1**
(45) Veröffentlichungstag und Bekanntmachung des Hinweises auf die Patenterteilung: **02.07.2008**
(21) Anmeldenummer: 02090141.9
(22) Anmeldetag: 09.03.1999
(51) Int. Cl.: C12N 15/12, C07K 14/47, C12N 15/62, C12N 5/10, C12N 1/21, C07K 16/30, G01N 33/50, G01N 33/68, A61K 38/17, A61P 35/00

(54) **Menschliche Nukleinsäuresequenz aus Prostatatumorgewebe**
Human nucleic acid sequence from prostate tumour tissue
Séquence d'acide nucléique humaine issue de tissus tumoraux prostatiques

(30) Priorität: 10.03.1998 DE 19811193
(43) Veröffentlichungstag der Anmeldung: 28.08.2002
(62) Teilanmeldung aus: 99917785.0
(73) Patentinhaber: Hinzmann, Bernd, Dr., 13127 Berlin (DE); Rosenthal, André, Prof., 14482 Potsdam (DE); Specht, Thomas, Dr., 83088 Kiefersfelden (DE); Pilarsky, Christian, Dr., 01309 Dresden (DE)
(72) Erfinder: Specht, Thomas, 14169 Berlin (DE); Hinzmann, Bernd, 13127 Berlin (DE); Schmitt, Armin, 96149 Breitengussbach (DE); Pilarsky, Christian, 14532 Stahnsdorf (DE); Dahl, Edgard, 14480 Postdam (DE); Rosenthal, André, 10115 Berlin (DE)

(56) Entgegenhaltungen:
- WO-A-97/36535
- WO-A-98/04689
- WO-A-99/09166

## Beschreibung

Die Erfindung betrifft menschliche Nukleinsäuresequenzen -mRNA, cDNA, genomische Sequenzen- aus Gewebe von Prostatatumoren, die für Genprodukte oder Teile davon kodieren und deren Verwendung.
Die Erfindung betrifft weiterhin die über die Sequenzen erhältlichen Polypeptide und deren Verwendung.

Eine weit verbreitete Krebsart ist der Prostatakrebs, für dessen Bekämpfung neue Therapien notwendig sind. Bisher verwendete Therapien, die auf einer Blockierung von Hormonwirkungen beruhen, sind sehr häufig nach wenigen Jahren wirkungslos, da der Tumor hormonunabhängig wird, d. h. ohne Hormonwirkung weiterwächst und Metastasen bildet.

Das Phänomen Krebs geht häufig einher mit der Über- oder Unterexpression gewisser Gene in den entarteten Zellen, wobei noch unklar ist, ob diese veränderten Expressionsraten Ursache oder Folge der malignen Transformation sind. Die Identifikation solcher Gene wäre ein wesentlicher Schritt für die Entwicklung neuer Therapien gegen Krebs. Der spontanen Entstehung von Krebs geht häufig eine Vielzahl von Mutationen voraus. Diese können verschiedenste Auswirkungen auf das Expressionsmuster in dem betroffenen Gewebe haben, wie z.B. Unter- oder Überexpression, aber auch Expression verkürzter Gene. Mehrere solcher Veränderungen durch solche Mutationskaskaden können schließlich zu bösartigen Entartungen führen. Die Komplexität solcher Zusammenhänge erschwert die experimentelle Herangehensweise sehr.

Für die Suche nach Tumor-bezogenen Kandidatengenen, d.h. Genen, die als Ursache für oder als Folge von bösartigen Entartungen normalen, menschlichen

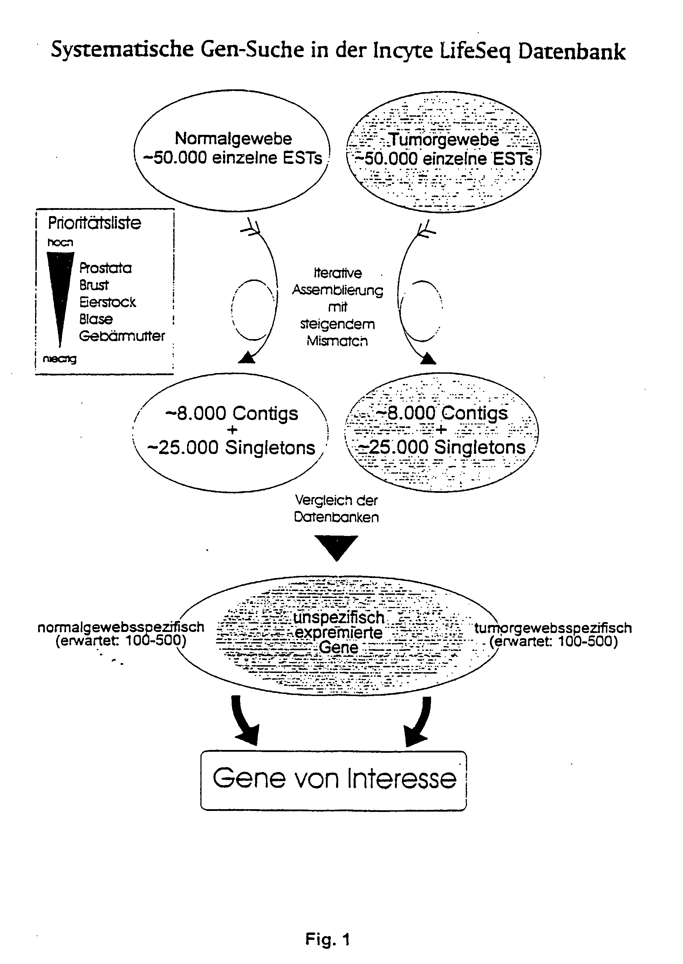

Gewebes angesehen werden können, wird eine Datenbank verwendet, die aus sogenanten ESTs besteht. ESTs (Expressed Sequence Tags) sind Sequenzen von cDNAs, d.h. revers transkribierten mRNAs, den Molekülen also, die die Expression von Genen widerspiegeln. Die EST-Sequenzen werden für normale und entartete Gewebe ermittelt. Solche Datenbanken werden von verschiedenen Betreibern z.T. kommerziell angeboten. Die ESTs der LifeSeq-Datenbank, die hier verwendet wird, sind in der Regel zwischen 150 und 350 Nukleotide lang. Sie representieren ein für ein bestimmtes Gen unverkennbares Muster, obwohl dieses Gen normalerweise sehr viel länger ist ( > 2000 Nukleotide). Durch Vergleich der Expressionsmuster von normalen und Tumorgewebe können ESTs identifiziert werden, die für die Tumorentstehung und -prolifertion wichtig sind (s. Fig. 1). Es besteht jedoch folgendes Problem: Da durch unterschiedliche Konstruktionen der cDNA-Bibliotheken die gefundenen EST-Sequenzen zu unterschiedlichen Regionen eines unbekannten Gens gehören können, ergäbe sich in einem solchen Fall ein völlig falsches Verhältnis des Vorkommens dieser ESTs in dem jeweiligen Gewebe. Dieses würde erst bemerkt werden, wenn das vollständige Gen bekannt ist und somit die ESTs dem gleichen Gen zugeordnet werden können.

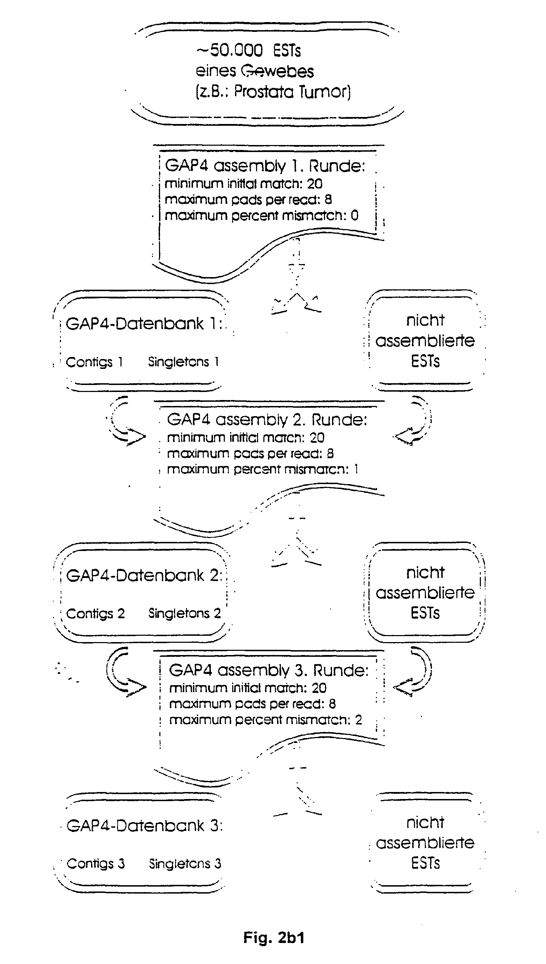

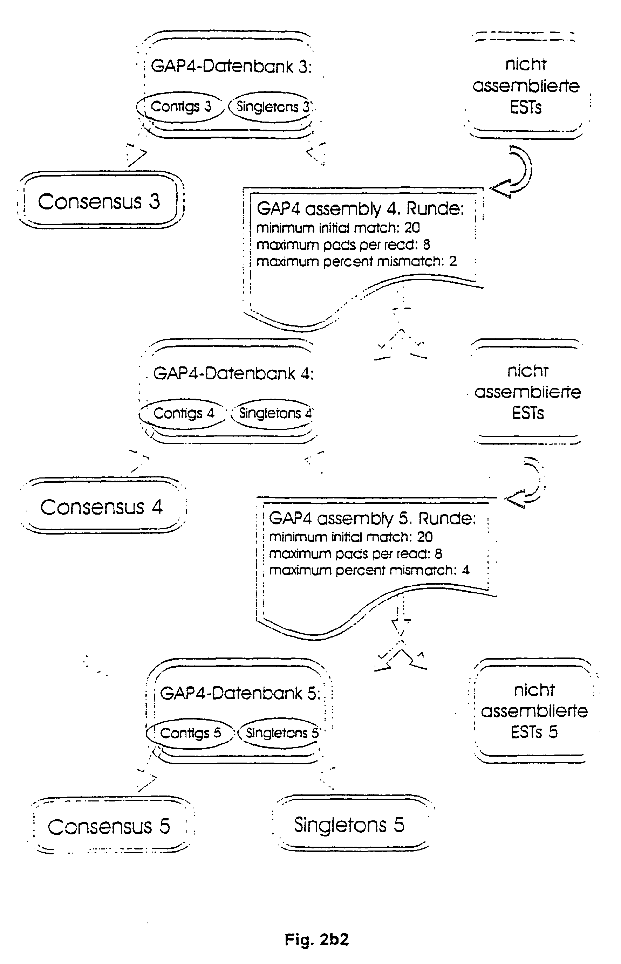

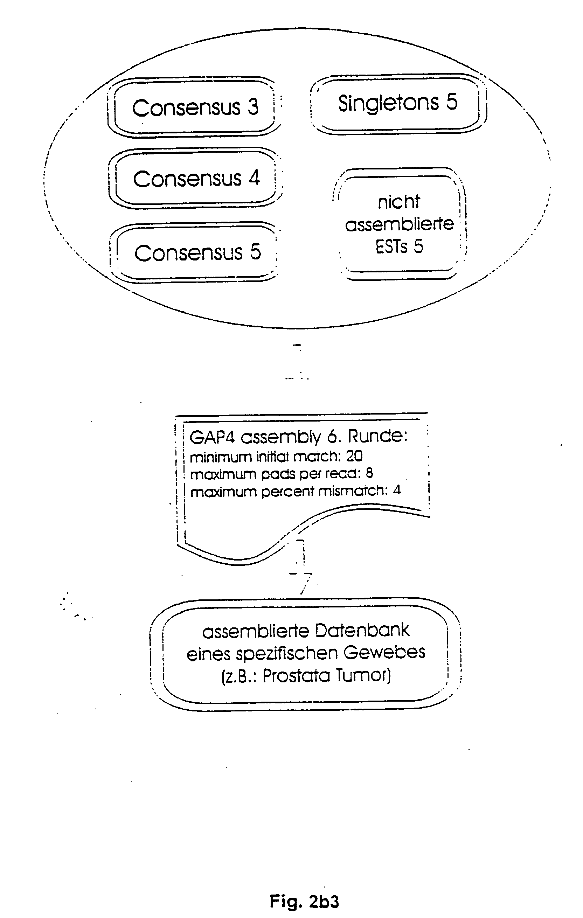

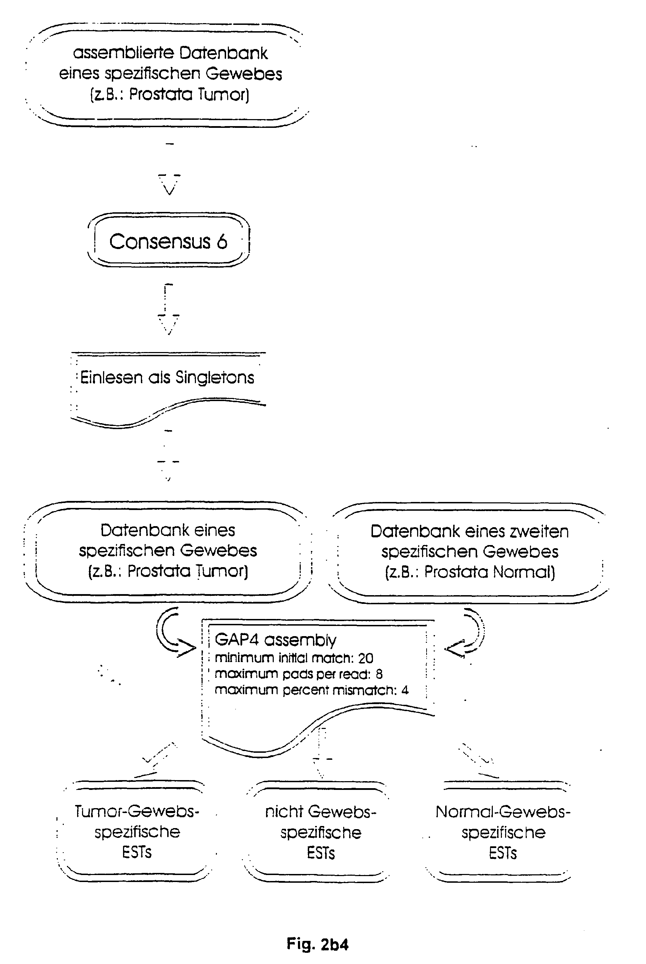

Es wurde nun gefunden, daß diese Fehlermöglichkeit verringert werden kann, wenn zuvor sämtliche ESTs aus dem jeweiligen Gewebstyp assembliert werden, bevor die Expressionsmuster miteinander verglichen werden. Es wurden also überlappende ESTs ein und desselben Gens zu längeren Sequenzen zusammengefaßt (s. Fig. 1, Fig. 2a und Fig. 3). Durch diese Verlängerung und damit Abdeckung eines wesentlich größeren Genbereichs in jeder der jeweiligen Banken sollte der oben beschriebene Fehler weitgehenst vermieden werden. Da es hierzu keine bestehenden Softwareprodukte gab, wurden Programme für das Assemblieren von genomischen Abschnitten verwendet, die abgewandelt eingesetzt und durch eigene Programme ergänzt wurden. Ein Flowchart der Assemblierungsprozedur ist in Fig. 2b1 - 2b4 dargestellt.

Es konnte nun die Nukleinsäure-Sequenz Seq. ID No. 50 gefunden werden, die als Kandidatengen bei Prostatakrebs eine Rolle spielen.

Mit der erfindungsgemäßen Teilsequenz Seq. ID No. 50 kann gemäß gängiger Verfahrenspraxis auch eine Expressionskassette konstruiert werden, wobei auf der Kassette mindestens eine erfindungsgemäße Nukleinsäure-Sequenz zusammen mit mindestens einer dem Fachmann allgemein bekannten Kontroll- oder regulatorischen Sequenz, wie z. B. einem geeigneten Promotor, kombiniert wird. Die erfindungsgemäßen Sequenzen können in sense oder antisense Orientierung eingefügt sein.

In der Literatur sind ist eine große Anzahl von Expressionskassetten bzw. Vektoren und Promotoren bekannt, die verwendet werden können.

Unter Expressionskassetten bzw. Vektoren sind zu verstehen: 1. bakterielle, wie z, B., phagescript, pBs, φX174, pBluescript SK, pBs KS, pNH8a, pNH16a, pNH18a, pNH46a (Stratagene), pTrc99A, pKK223-3, pKK233-3, pDR540, pRIT5 (Pharmacia), 2. eukaryontische, wie z. B. pWLneo, pSV2cat, pOG44, pXT1, pSG (Stratagene), pSVK3, pBPV, pMSG, pSVL (Pharmacia).

Unter Kontroll- oder regulatorischer Sequenz sind geignete Promotoren zu verstehen. Hierbei sind zwei bevorzugte Vektoren der pKK232-8 und der PCM7 Vektor. Im einzelnen sind folgende Promotoren gemeint: lacl, lacZ, T3, T7, gpt, lambda P_{R}, trc, CMV, HSV Thymidin-Kinase, SV40, LTRs aus Retrovirus und Maus Metallothionein-I.

Die auf der Expressionskassette befindlichen DNA-Sequenzen können ein Fusionsprotein kodieren, das ein bekanntes Protein und ein biologisch aktives Polypeptid-Fragment umfaßt.

Die Expressionskassetten sind ebenfalls Gegenstand der vorliegenden Erfindung.

Die erfindungsgemäßen Nukleinsäure-Fragmente können zur Herstellung von Vollängen-Genen verwendet werden. Die erhältlichen Gene sind ebenfalls Gegenstand der vorliegenden Erfindung.

Die Erfindung betrifft auch die Verwendung der erfindungsgemäßen Nukleinsäure-Sequenzen, sowie die aus der Verwendung erhältlichen Gen-Fragmente.

Die erfindungsgemäßen Nukleinsäure-Sequenzen können mit geeigneten Vektoren in Wirtszellen gebracht werden, in denen als heterologer Teil die auf den Nukleinsäure-Fragmenten enthaltene genetischen Information befindet, die exprimiert wird.

Die die Nukleinsäure-Fragmente enthaltenden Wirtszellen sind ebenfalls Gegenstand der vorliegenden Erfindung.

Geeignete Wirtszellen sind z. B. prokaryontische Zellsysteme wie E. coli oder eukaryontische Zellsysteme wie tierische oder humane Zellen oder Hefen.

Die erfindungsgemäßen Nukleinsäure-Sequenzen können in sense oder antisense Form verwendet werden.

Die Herstellung der Polypeptide oder deren Fragment erfolgt durch Kultivierung der Wirtszellen gemäß gängiger Kultivierungsmethoden und anschließender Isolierung und Aufreinigung der Peptide bzw. Fragmente, ebenfalls mittels gängiger Verfahren.

Die gefundenen erfindungsgemäßen Nukleinsäure-Sequenzen können auch genomische oder mRNA-Sequenzen sein.

Die Erfindung betrifft auch genomische Gene, ihre Promotoren, Enhancer, Silencer, Exonstruktur, Intronstruktur und deren Spleißvarianten, erhältlich aus den cDNAs der Sequenz Seq. ID No. 50, sowie deren Verwendung zusammen mit geeigneten regulativen Elementen, wie geeigneten Promotoren und/ oder Enhancern.

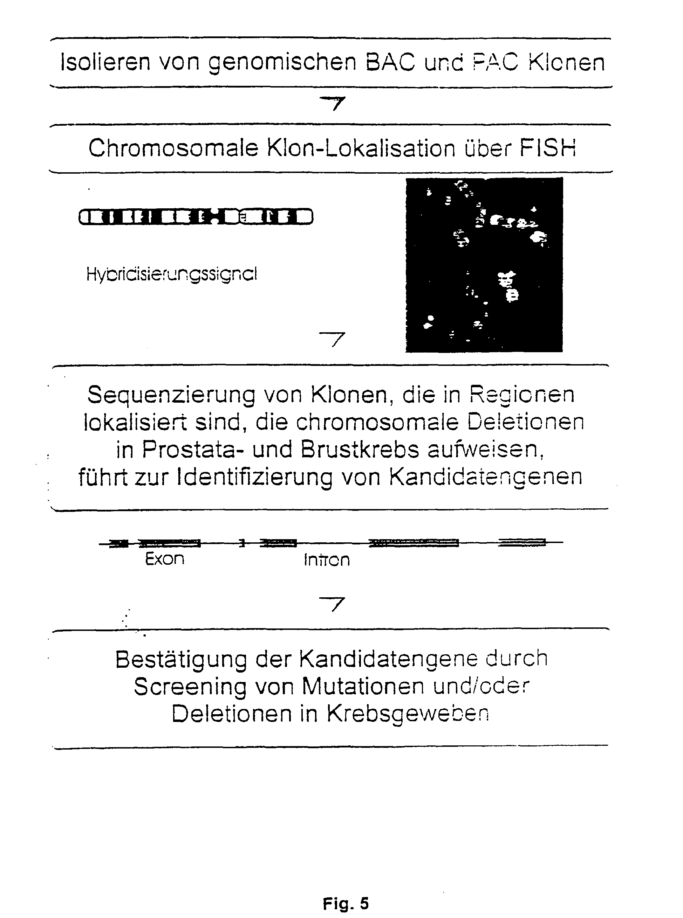

Mit den erfindungsgemäßen Nukleinsäuren (cDNA-Sequenzen) werden genomische BAC-, PAC- und Cosmid-Bibliotheken gescreent und über komplementäre Basenpaarung (Hybridisierung) spezifisch humane Klone isoliert. Die so isolierten BAC-, PAC- und Cosmid-Klone werden mit Hilfe der Fluoreszenz-in-situ-Hybridisation auf Metaphasenchromosomen hybridisiert und entsprechende Chromosomenabschnitte identifiziert, auf denen die entsprechenden genomischen Gene liegen. BAC-, PAC- und Cosmid-Klone werden sequenziert, um die entsprechenden genomischen Gene in ihrer vollständigen Struktur (Promotoren, Enhancer, Silencer, Exons und Introns) aufzuklären. BAC-, PAC- und Cosmid-Klone können als eigenständige Moleküle für den Gentransfer eingesetzt werden (s. Fig. 5).

### Bedeutungen von Fachbegriffen und Abkürzungen

| | |
|---|---|
| Nukleinsäuren= | Unter Nukleinsäuren sind in der voliegenden Erfindung zu verstehen: mRNA, partielle cDNA, vollängen cDNA und genomische Gene (Chromosomen). |
| ORF = | Open Reading Frame, eine definierte Abfolge von Aminosäuren, die von der cDNA-Sequenz abgeleitet werden kann. |
| Contig= | Eine Menge von DNA-Sequenzen, die aufgrund sehr großer Ähnlichkeiten zu einer Sequenz zusammengefaßt werden können (Consensus). |
| Singleton= | Ein Contig, der nur eine Sequenz enthält. |
| Modul = | Domäne eines Proteins mit einer definierten Sequenz, die eine strukturelle Einheit darstellt und in unterschiedlichen Proteinen vorkommt |
| N = | wahlweise das Nukleotid A, T, G oder C |
| X = | wahlweise eine der 20 natürlich vorkommenden Aminosäuren |

### Erklärung zu den Alignmentparametern

| | |
|---|---|
| minimal initial match= | minimaler anfänglicher Identitätsbereich |
| maximum pads per read= | maximale Anzahl von Insertionen |
| maximum percent mismatch= | maximale Abweichung in % |

### Erklärung der Abbildungen

- Fig. 1: zeigt die systematische Gen-Suche in der Incyte LifeSeq Datenbank.
- Fig. 2a: zeigt das Prinzip der EST-Assemblierung
- Fig. 2b1-2b4: zeigt das gesamte Prinzip der EST-Assemblierung
- Fig. 3: zeigt die in silico Subtraktion der Genexpression in verschiedenen Geweben
- Fig. 4a: zeigt die Bestimmung der gewebsspezifischen Expression über elektronischen Northern.
- Fig. 4b: zeigt den elektronischen Northern
- Fig. 5: zeigt die Isolierung von genomischen BAC- und PAC-Klonen.

Die nachfolgenden Beispiele erläutern die Herstellung der erfindungsgemäßen Nukleinsäure-Sequenzen, ohne die Erfindung auf diese Beispiele und Nukleinsäure-Sequenzen zu beschränken.

### Beispiel 1

### Suche nach Tumor-bezogenen Kandidatengenen

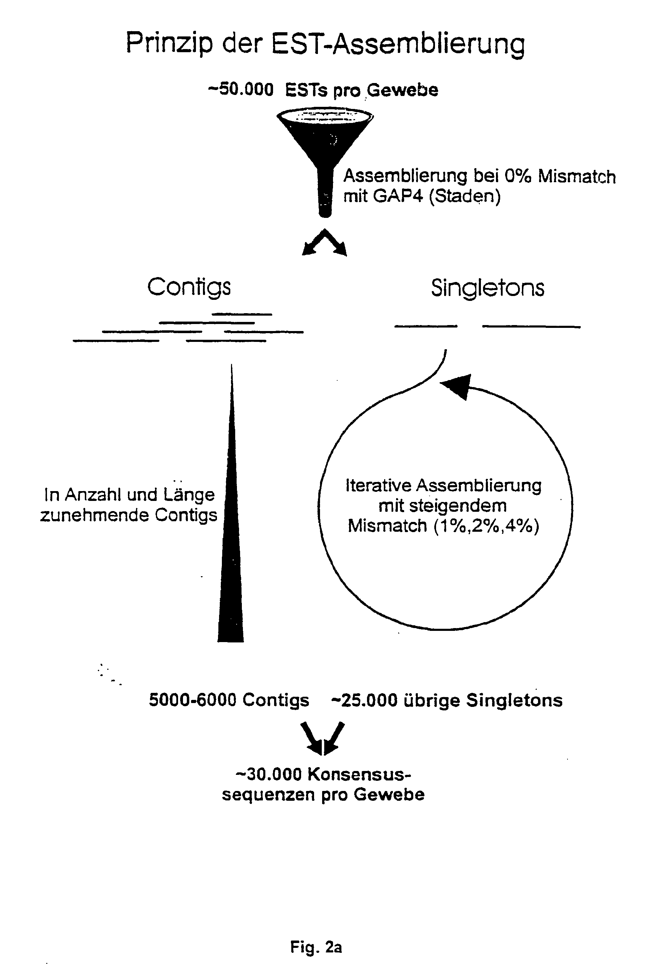

Zuerst wurden sämtliche ESTs des entsprechenden Gewebes aus der LifeSeq-Datenbank (vom Oktober 1997) extrahiert. Diese wurden dann mittels des Programms GAP4 des Staden-Pakets mit den Parametern 0% mismatch, 8 pads per read und einem minimalen match von 20 assembliert. Die nicht in die GAP4-Datenbank aufgenommenen Sequenzen (Fails) wurden erst bei 1 % mismatch und dann nochmals bei 2% mismatch mit der Datenbank assembliert. Aus den Contigs der Datenbank, die aus mehr als einer Sequenz bestanden, wurden Consensussequenzen errechnet (s. Fig. 2a und 2 b1-2b4).

Die Singletons der Datenbank, die nur aus einer Sequenz bestanden, wurden mit den nicht in die GAP4-Datenbank aufgenommenen Sequenzen bei 2% mismatch erneut assembliert. Wiederum wurden für die Contigs die Consensussequenzen ermittelt. Alle übrigen ESTs wurden bei 4% mismatch erneut assembliert. Die Consensussequenzen wurden abermals extrahiert und mit den vorherigen Consensussequenzen sowie den Singletons und den nicht in die Datenbank aufgenommenen Sequenzen abschließend bei 4% mismatch assembliert. Die Consensussequenzen wurden gebildet und mit den Singletons und Fails als Ausgangsbasis für die Gewebsvergleiche verwendet. Durch diese Prozedur konnte sichergestellt werden, daß unter den verwendeten Parametern sämtliche Sequenzen von einander unabhängige Genbereiche darstellten.

Fig. 2b1-2b4 veranschaulicht die Verlängerung der Prostata-Tumor ESTs.

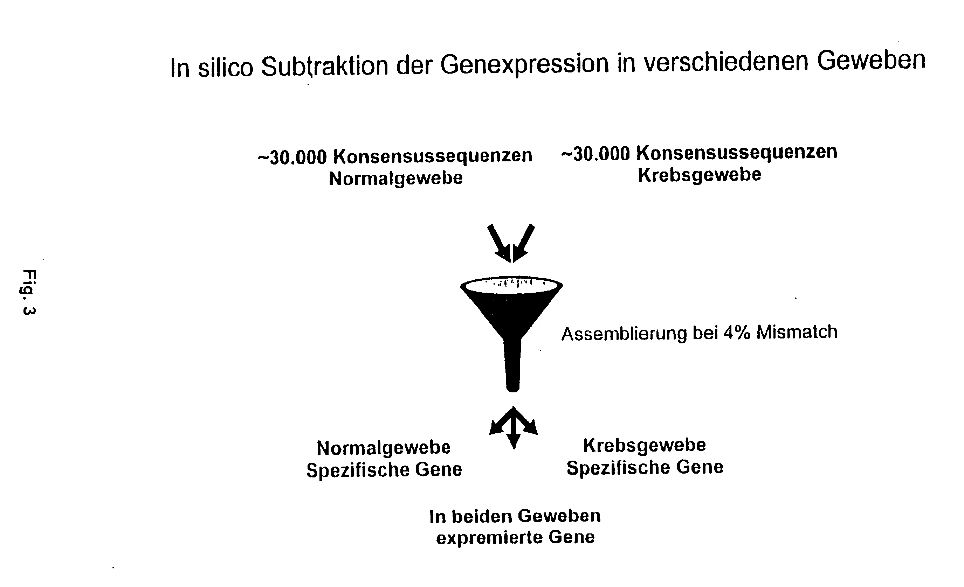

Die so assemblierten Sequenzen der jeweiligen Gewebe wurden anschließend mittels des gleichen Programms miteinander verglichen (s. Fig. 3). Hierzu wurden erst alle Sequenzen des ersten Gewebes in die Datenbank eingegeben. (Daher war es wichtig, daß diese voneinander unabhängig waren.)
Dann wurden alle Sequenzen des zweiten Gewebes mit allen des ersten verglichen. Das Ergebnis waren Sequenzen, die für das erste bzw. das zweite Gewebe spezifisch waren, sowie welche, die in beiden vorkamen. Bei Letzteren wurde das Verhältnis der Häufigkeit des Vorkommens in den jeweiligen Geweben ausgewertet.

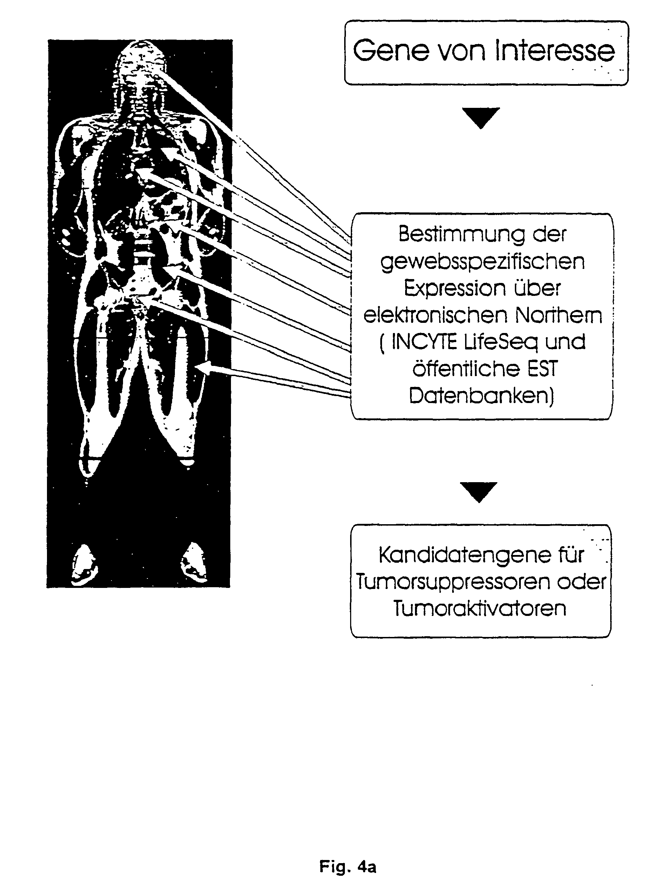

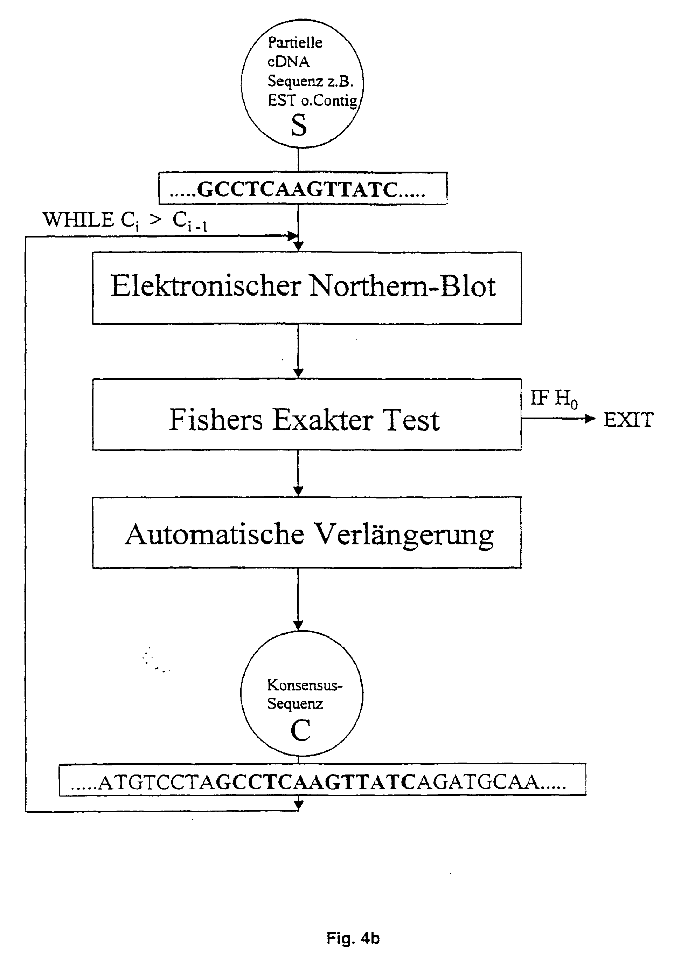

Alle Sequenzen, die mehr als viermal in jeweils einem der verglichenen Gewebe vorkamen, sowie alle, die mindestens fünfmal so häufig in einem der beiden Gewebe vorkamen wurden weiter untersucht. Diese Sequenzen wurden einem elektronischen Northern (s. Beispiel 2.1) unterzogen, wodurch die Verteilung in sämtlichen Tumor- und Normal-Geweben untersucht wurde (s. Fig. 4a und Fig. 4b). Die relevanten Kandidaten wurden dann mit Hilfe sämtlicher Incyte ESTs und allen ESTs öffentlicher Datenbanken verlängert (s. Beispiel 3). Anschließend wurden die Sequenzen und ihre Übersetzung in mögliche Proteine mit allen Nukleotid- und Proteindatenbanken verglichen, sowie auf mögliche, für Proteine kodierende Regionen untersucht.

### Beispiel 2

### Algorithmus zur Identifikation und Verlängerung von partiellen cDNA-Sequenzen mit verändertem Expressionsmuster

Im folgenden soll ein Algorithmus zur Auffindung über- oder unterexprimierter Gene erläutert werden. Die einzelnen Schritte sind der besseren Übersicht halber auch in einem Flußdiagramm zusammengefaßt (s. Fig. 4b).

### 2.1 Elektronischer Northern-Blot

Zu einer partiellen DNA-Sequenz S, z. B. einem einzelnen EST oder einem Contig von ESTs, werden mittels eines Standardprogramms zur Homolgiesuche, z. B. BLAST (Altschul, S. F., Gish W., Miller, W., Myers, E. W. und Lipman, D. J. (1990) J. Mol. Biol., 215, 403-410), BLAST2 (Altschul, S. F., Madden, T. L., Schäffer, A. A., Zhang, J., Zhang, Z., Miller, W. und Lipman, D. J. (1997) Nucleic Acids Research 25 3389-3402) oder FASTA (Pearson, W. R. und Lipman, D. J. (1988) Proc. Natl. Acad. Sci. USA 85 2444-2448), die homologen Sequenzen in verschiedenen nach Geweben geordneten (privaten oder öffentlichen) EST-Bibliotheken bestimmt. Die dadurch ermittelten (relativen oder absoluten) Gewebespezifischen Vorkommenshäufigkeiten dieser Partial-Sequenz S werden als elektronischer Northern-Blot bezeichnet.

### 2.1.1

Analog der unter 2.1 beschriebenen Verfahrensweise wurde das EST der Sequenz Seq. ID No. 50 gefunden.

Das Ergebnis ist wie folgt:

| Elektronischer Northern-Blot für SEQ. ID. NO: 50 | | | | |
|---|---|---|---|---|
| | NORMAL | TUMOR | Verhaeltnisse | |
| | %Haeufigkeit | %Haeufigkeit | N/T | T/N |
| Blase | 0.0000 | 0.0000 | undef | undef |
| Brust | 0.0000 | 0.0000 | undef | undef |
| Eierstock | 0.0000 | 0.0000 | undef | undef |
| Endokrines_Gewebe | 0.0000 | 0.0000 | undef | undef |
| Gastrointestinal | 0.0000 | 0.0000 | undef | undef |
| Gehirn | 0.0000 | 0.0000 | undef | undef |
| Haematopoetisch | 0.0000 | 0.0000 | undef | undef |
| Haut | 0.0000 | 0.0000 | undef | undef |
| Hepatisch | 0.0000 | 0.0000 | undef | undef |
| Herz | 0.0000 | 0.0000 | undef | undef |
| Hoden | 0.0000 | 0.0000 | undef | undef |
| Lunge | 0.0000 | 0.0000 | undef | undef |
| Magen-Speiseroehre | 0.0000 | 0.0000 | undef | undef |
| Muskel-Skelett | 0.0000 | 0.0000 | undef | undef |
| Niere | 0.0000 | 0.0000 | undef | undef |
| Pankreas | 0.0000 | 0.0000 | undef | undef |
| Penis | 0.0000 | 0.0000 | undef | undef |
| Prostata | 0.0024 | 0.0255 | 0.0932 | 10.7274 |
| Uterus | 0.0000 | 0.0000 | undef | undef |
| Brust-Hyperplasie | 0.0000 | | | |
| Duenndarm | 0.0000 | | | |
| Prostata-Hyperplasie | 0.0030 | | | |
| Samenblase | 0.0000 | | | |
| sinnesorgane | 0.0000 | | | |
| Weisse_Blutkoerperchen | 0.0000 | | | |
| | | | | |

| | FOETUS | | | |
|---|---|---|---|---|
| | %Haeufigkeit | | | |
| Entwicklung | 0.0000 | | | |
| Gastrointenstinal | 0.0000 | | | |
| Gehirn | 0.0000 | | | |
| Haematopoetisch | 0.0000 | | | |
| Herz-Blutgefaesse | 0.0000 | | | |
| Lunge | 0.0000 | | | |
| Niere | 0.0000 | | | |
| Prostata | 0.0000 | | | |
| Sinnesorgane | 0.0000 | | | |
| | | | | |

| NORMIERTE/SUBTRAHIERTE BIBLIOTHEKEN | | | | |
|---|---|---|---|---|
| | %Haeufigkeit | | | |
| Brust | 0.0000 | | | |
| Eierstock-Uterus | 0.0000 | | | |
| Endokrines_Gewebe | 0.0000 | | | |
| Foetal | 0.0000 | | | |
| Gastrointestinal | 0.0000 | | | |
| Haematopoetisch | 0.0000 | | | |
| Haut-Muskel | 0.0000 | | | |
| Hoden | 0.0000 | | | |
| Lunge | 0.0000 | | | |
| Nerven | 0.0000 | | | |
| Prostata | 0.0000 | | | |
| Sinnesorgane | 0.0000 | | | |

### 2.2 Fisher-Test

Um zu entscheiden, ob eine Partial-Sequenz S eines Gens in einer Bibliothek für Normal-Gewebe signifikant häufiger oder seltener vorkommt als in einer Bibliothek für entartetes Gewebe, wird Fishers Exakter Test, ein statistisches Standardverfahren (Hays, W. L., (1991) Statistics, Harcourt Brace College Publishers, Fort Worth), durchgeführt.
Die Null-Hypothese lautet: die beiden Bibliotheken können bezüglich der Häufigkeit zu S homologer Sequenzen nicht unterschieden werden. Falls die Null-Hypothese mit hinreichend hoher Sicherheit abgelehnt werden kann, wird das zu S gehörende Gen als interessanter Kandidat für ein Krebs-Gen akzeptiert, und es wird im nächsten Schritt versucht, eine Verlängerung seiner Sequenz zu erreichen.

### Beispiel 3

### Automatische Verlängerung der Partial-Sequenz

Die automatische Verlängerung der Partial-Sequenz S vollzieht sich in drei Schritten:
1. Ermittlung aller zu *S* homologen Sequenzen aus der Gesamtmenge der zur Verfügung stehenden Sequenzen mit Hilfe von BLAST
2. Assemblierung dieser Sequenzen mittels des Standardprogramms GAP4 (Bonfield, J. K., Smith, K. F., und Staden R. (1995), Nucleic Acids Research 23 4992-4999) (Contig-Bildung).
3. Berechnung einer Konsens-Sequenz *C* aus den assemblierten Sequenzen

Die Konsens-Sequenz *C* wird im allgemeinen länger sein als die Ausgangssequenz *S*. Ihr elektronischer Northern-Blot wird demzufolge von dem für *S* abweichen. Ein erneuter Fisher-Test entscheidet, ob die Alternativ-Hypothese der Abweichung von einer gleichmäßigen Expression in beiden Bibliotheken aufrechterhalten werden kann. Ist dies der Fall, wird versucht, *C* in gleicher Weise wie *S* zu verlängern. Diese Iteration wird mit der jeweils erhaltenen Konsensus-Sequenzen *Cᵢ* (*i*: Index der Iteration) fortgesetzt, bis die Alternativ-Hypothese verworfen wird (if H₀ Exit; Abbruchkriterium I) oder bis keine automatische Verlängerung mehr möglich ist (while *Cᵢ* > *Cᵢ₋₁*; Abbruchkriterium II).

Im Fall des Abbruchkriteriums II bekommt man mit der nach der letzten Iteration vorliegenden Konsens-Sequenz eine komplette oder annähernd komplette Sequenz eines Gens, das mit hoher statistischer Sicherheit mit Krebs in Zusammenhang gebracht werden kann.

Analog der oben beschriebenen Beispiele konnten die in der Tabelle I beschriebenen Nukleinsäure-Sequenzen aus Prostatatumor-Gewebe gefunden werden.

### Beispiel 4

### Kartierung der Nukleinsäure-Sequenzen auf dem humanen Genom

Die Kartierung der humanen Gene erfolgte unter Verwendung des Stanford G3 Hybrid-Panels (Stewart et al., 1997), der von Research Genetics, Huntsville, Alabama vertrieben wird. Dieses Panel besteht aus 83 verschiedenen genomischen DNAs von Mensch-Hamster Hybridzellinien und erlaubt eine Auflösung von 500 Kilobasen. Die Hybridzellinien wurden durch Fusion von bestrahlten diploiden menschlichen Zellen mit Zellen des Chinesischen Hamsters gewonnen. Das Rückhaltemuster der humanen Chromosomenfragmente wird mittels genspezifischer Primer in einer Polymerase-Kettenreaktion bestimmt und mit Hilfe der vom Stanford RH Server verfügbaren Software analysiert (http://www.stanford.edu/RH/rhserver_ form2.html). Dieses Programm bestimmt den STS-Marker, der am nächsten zum gesuchten Gen liegt. Die entsprechende zytogenetische Bande wurde unter Verwendung des "Mapview" -Programms der Genome Database (GDB), (http://gdbwww.dkfz-heidelberg.de) bestimmt.
Neben dem kartieren von Genen auf dem menschlichen Cromosomensatz durch verschiedene experimentelle Methoden ist es möglich die Lage von Genen auf diesem durch bioinformatische Methoden zu bestimmen. Dazu wurde das bekannte Programm e-PCR eingesetzt (Schuler GD (1998) Electronic PCR: bridging the gap between genome mapping and genome sequencing. Trends Biotechnol 16; 456-459, Schuler GD (1997). Sequence mapping by electronic PCR. Genome Res 7; 541-550). Die dabei eingesetzte Datenbank entspricht nicht mehr der in der Literatur angegebenen, sonder ist eine Weiterentwicklung, welche Daten der öffentlichen Datenbank RHdb (http://www.ebi.ac.uk/RHdb/index.html) einschließt. Analog zu der Kartierung durch die Hybrid-Panels erfolgte eine Auswertung der Ergebnisse mit der obengenannten Software und der Software des Whitehead-Institutes (http://carbon.wi.mit.edu:8000/cgi-bin/contig/rhmapper.pl).

### Beispiel 5

### Gewinnung von genomischen DNA-Sequenzen (BAC-Klone)

Die die entsprechenden cDNA enthaltenen genomischen BAC-Klone (http://www.tree.caltech.edu/; Shizuya, H., B. Birren, U-J. Kim, V. Mancino, T. Slepak, Y. Tachiiri, M. Simon (1992) Proc. Natl. Acad. Sci., USA 89: 8794-8797) wurden mit der Prozedur des "down-to-the-well" isoliert. Bei dieser Prozedur wird eine Bibliothek bestehend aus BAC-Klonen (die Bibliothek überdeckt ca. 3 x das humane Genom) in ein bestimmtes Raster gebracht, so daß die DNA dieser Klone mit einer spezifischen PCR untersucht werden kann. Dabei erfolgt ein "Poolen" der DNA verschiedener BAC-Klone. Durch eine kombinatorische Analyse ist es möglich die Klone zu bestimmen, die die gesuchte DNA enthalten. Durch das Festlegen der Klone kann die Adresse der Klone in der Bibliothek bestimmt werden. Diese Adresse zusammen mit dem Namen der verwendeten Bibliothek legen die Klone und damit die DNA-Sequenz dieser Klone eindeutig fest.
Die nachfolgenden Beispiele erläutern die erfolgreiche Isolierung der genomischen BAC-Klone ohne, diese darauf zu beschränken. Die verwendete Bibliotheken waren CITB B und CITB C:

| Seq. ID Nr. 50 | Identifizierte BACs | | | | |
|---|---|---|---|---|---|
| 50 | 423-P-13 | 327-G-1 | 221-M-24 | 197-M-19 | |

**Tabelle 1**

| Seq ID | Expression | Funktion | Länge des Ausgangs-EST in Basen | Länge der beanspruchten Sequenz in Basen | Seq ID der Ausgangs-sequenz |
|---|---|---|---|---|---|
| 50 | In Prostatatumoren erhöht | Unbekannt | 277 | 649 | |

### Sequenzprotokoll

(2) INFORMATION ÜBER SEQ ID NO: 50:
   (i) SEQUENZ CHARAKTERISTIK:
      (A) LÄNGE: 649 Basenpaare
      (B) TYP: Nukleinsäure
      (C) STRANG: einzel
      (D) TOPOLOGIE: linear
   (ii) MOLEKÜLTYP: Aus einzelnen ESTs durch Assemblierung und Editierung hergestellte partielle cDNAs
   (iii) HYPOTHETISCH: NEIN
   (iii) ANTI-SENSE: NEIN
   (vi) HERKUNFT:
      (A) ORGANISMUS: MENSCH
      (C) ORGAN:
   (vii) SONSTIGE HERKUNFT:
      (A) BIBLIOTHEK: cDNA library
   (xi) SEQUENZ-BESCHREIBUNG: SEQ ID NO: 50:

agatattatg gatggtgaag ggaatggtat agaattggag agattatctt actgaacacc 60
tgtagtccca gctttctctg gaagtggtgg tatttgagca ggatgtgcac aaggcaattg120
aaatgcccat aattagtttc tcagctttga atacactata aactcagtgg ctgaaggagg180
aaattttaga aggaagctac taaaagatct aatttgaaaa actacaaaag cattaactaa240
aaaagtttat tttccttttg tctgggcagt agtgaaaata actactcaca acattcacta300
tgtttgcgag ggattaacac aaataaaaga tgccttttta cttaaacacc aagacagaga360
acttgcccaa tactgagaag caacttccac tagagaggga actgttaaat gttttcaacc420
cagttcatct ggtggatgtt tttgcaggtt actctgagaa ttttgcttat gaaaaatcat480
tatttttagt gtagttcaca ataatgtatt gaacatactt ctaatcaaag gtgctatgtc540
cttgtgtatg gtactaaatg tgtcctgtgt acttttgcac aactgagaat cctgcagctt600
ggtttaatga gtgtgttcat gaaataaata atggaggaat tgtcaaaaa

## Patentansprüche

1. Eine isolierte Nukleinsäure-Sequenz, die einen Teil eines Transkriptes darstellt, (a) umfassend eine Nukleinsäuresequenz Seq. ID No. 50, **dadurch gekennzeichnet, dass** die in Prostatatumorgewebe erhöht exprimiert ist, (b) oder eine Nukleinsäuresequenz, die Komplementär zu Seq. ID No. 50 ist.

2. Eine Nukleinsäuresequenz gemäß Anspruch 1, **dadurch gekennzeichnet, dass** sie transkribiert wird.

3. Verwendung der Nukleinsäure-Sequenzen gemäß Anspruch 1 zur Herstellung des Volllänge Gens.

4. Wirtzellen ausserhalb des menschlichen bzw. tierischen körpers, enthaltend als heterologen Teils ihrer transkribierbaren genetischen Information ein Nukleinsäurefragment gemäß Anspruch 1.

5. Nukleinsäure-Sequenz Seq ID No 50 in sense oder antisense Form.

6. Ein Molekül mit einer Nukleinsäure-Sequenz gemäß Anspruch 10, **dadurch gekennzeichnet, dass** sie eine entsprechende mRNA darstellt.

## Claims

1. An isolated nucleic acid sequence representing a part of a transcript,
a) comprising a nucleic acid sequence Seq. ID No. 50, **characterized by** that it is expressed to an increased degree in prostate tumor tissue,
b) or a nucleic acid sequence, which is complementary to Seq. ID No. 50.

2. A nucleic acid sequence according to claim 1, **characterized by** that it is transcribed.

3. The use of the nucleic acid sequences according to claim 1 for producing the full-length gene.

4. Host cells outside the human or animal body, comprising as a heterologous part of their transcribable genetic information a nucleic acid fragment according to claim 1.

5. The nucleic acid sequence SEQ ID 50 in sense or anti-sense form.

6. A molecule comprising a nucleic acid sequence according to claim 1 , **characterized by** that it represents a corresponding mRNA.

## Revendications

1. Séquence d'acide nucléique isolée représentant une partie d'un transcrit,
a) comprenant une séquence d'acide nucléique Séq. ID N° 50, **caractérisée en ce qu'**elle est exprimée à un degré augmenté dans le tissu de tumeur de la prostate,
b) ou une séquence d'acide nucléique, qui est complémentaire à Séq. ID N° 50.

2. Séquence d'acide nucléique selon la revendication 1, **caractérisée en ce qu'**elle est transcrite.

3. Utilisation de la séquence d'acide nucléique selon la revendication 1 pour la production d'un gène pleine longueur.

4. Cellules hôtes à l'extérieur du corps humain ou animal, comprenant comme partie hétérologique de leur information génétique transcrivable un fragment d'acide nucléique selon la revendication 1.

5. Séquence d'acide nucléique SEQ ID 50 en forme sens ou antisens.

6. Molécule comprenant une séquence d'acide nucléique selon la revendication 1, **caractérisée en ce qu'**elle représente un mARN correspondant.
